# EUROPEAN PATENT SPECIFICATION

(11) **EP 1 608 971 B1**
(45) Date of publication and mention of the grant of the patent: **13.02.2008**
(21) Application number: 03719079.0
(22) Date of filing: 31.03.2003
(51) Int. Cl.: G01N 33/543, C12Q 1/68

(54) **RAPID HEAT-MEDIATED METHOD FOR ENZYME-LINKED IMMUNOSORBENT ASSAY PROCEDURE**
SCHNELLES, HITZE-VERMITTELTES VERFAHREN FÜR ELISA
PROCEDE RAPIDE UTILISANT LA CHALEUR POUR PROCEDURE DE DOSAGE IMMUNOENZYMATIQUE

(43) Date of publication of application: 28.12.2005
(73) Proprietor: COUNCIL OF SCIENTIFIC AND INDUSTRIAL RESEARCH, New Delhi 110 001 (IN)
(72) Inventor: NAHAR, Pradip, Mall Road, Delhi 110 007 (IN); BORA, Utpal, Mall Road, Delhi 110 007 (IN)
(74) Representative: Smyth, Gyles Darren
(86) International application number: PCT/IN2003/000103
(87) International publication number: WO 2004/088315

(56) References cited:
- EP-A- 0 058 428
- EP-A- 0 236 069
- EP-A- 0 857 974
- WO-A-89/03038
- US-B1- 6 498 016
- LABROUSSE H ET AL: "Effect of temperature on the reactivities of polyreactive and monospecific monoclonal IgG antibodies" RESEARCH IN IMMUNOLOGY, vol. 148, no. 4, 1997, pages 267-276, XP002269723 ISSN: 0923-2494
- DORP VAN R ET AL: "ELISA INCUBATION TIMES CAN BE REDUCED BY 2.45-GHZ MICROWAVES" JOURNAL OF CLINICAL AND LABORATORY IMMUNOLOGY, TREVIOT-KIMPTON PUBLICATIONS, LONDON, GB, vol. 34, no. 2, February 1991 (1991-02), pages 87-96, XP000985204 ISSN: 0141-2760
- DORP VAN R ET AL: "A RAPID ELISA FOR MEASUREMENT OF ANTI-GLOMERULAR BASEMENT MEMBRANE ANTIBODIES USING MICROWAVES" JOURNAL OF CLINICAL AND LABORATORY IMMUNOLOGY, TREVIOT-KIMPTON PUBLICATIONS, LONDON, GB, vol. 40, no. 3, 1993, pages 135-147, XP000985202 ISSN: 0141-2760

## Description

### Field of the invention

The present invention relates to a rapid and efficient method for carrying out enzyme-linked immunosorbent assay for detection of minute quantities of biomolecules such as antigen, antibody etc. This invention particularly relates to heat- mediated immobilization of antigen or antibody on to the activated surface followed by performing subsequent steps of ELISA by controlled temperature. The invented procedure has reduced the total time required for ELISA to around 3 h. The invented ELISA procedure is rapid, economical, reproducible and simple.

The invented procedure is useful for carrying out ELISA required in clinical diagnostics, molecular biology, agriculture, food technology, environmental science etc. The invented ELISA method is simple, time saving and obviates the time consuming procedure. This method has the potential for automation.

### Background of the invention

Enzyme linked immunosorbent assay (ELISA) is a very sensitive technique used for detection of certain antigens and antibodies. ELISA has become a useful tool in diagnosis of diseases in both animals and plants. ELISA can also be used for screening of monoclonal antibodies during the course of their production (Douillard, J.Y. and Hoffman, T., 1983), pesticide residue detection in crop produce (Van Emon, J.M. and Lopez-Avila, V., 1992) and environmental samples like soil and water (Ghassempour et al. 2002), detection of apoptosis in tissue culture etc. (Salgame, P. et al, 1996).

Conventional ELISA procedures carried out by immobilizing antigen or antibody on a polystyrene microtiter plate through adsorption have following drawbacks: (i) ELISA values in different wells and plates are usually inconsistent (Kricka et al., 1980; Hermann and Collins, 1976); (ii) long incubation times is required; (iii) during washing some biomolecules may detach leading to nonreproducible results (Engvall and Perlmann, 1971); and (iv) usually gives lower sensitivity (Kemeny, 1997). On the other hand, covalent binding is more sensitive (Deshpande, 1996), minimizes nonspecific binding and eliminates the above shortcomings (Douglas and Monteith, 1994). Covalent binding of immunogens to grafted plastic surfaces has been reported (Larsson, P.H. et al, 1987).

Aleixo et al. (1985), actived an inert polystyrene surface by nitrating the aromatic ring of the polystyrene followed by the reduction of the nitro group to an amino group. The amino polystyrene was further activated by chemical reactions such as diazotization and the resulting activated surface was used to immobilize antigen covalently.

Despite this, the conventional ELISA method requires very long time varying from several hours to 2 days for completion. This is the main drawback of different ELISA methods, based either on adsorption or on covalent binding. In case of medical urgency, precious time is lost in diagnosis before the patient could be given medication. In agriculture, ELISA is useful for detecting pesticide residues in crop produce (Van Emon, J.M. and Lopez-Avila, V., 1992) and environmental samples (Ghassempour et al. 2002).

Bora et al. (2002) have reported a rapid and simple method for a double-anti-body sandwich (DAS) ELISA and a direct antigen coating (DAC) ELISA by covalently immobilizing antigen or antibody onto the activated polystyrene surface which was developed by Nahar et al. (2001) by a rapid and simple method using 1-fluoro-2-nitro-4-azidobenzene (FNAB). Detection of antibodies at lower concentrations was also done successfully using the activated surface. The ELISA value obtained in eight hours by this method was found to be similar with the values obtained in the conventional ELISA method carried out in fifteen hours. Although this method had reduced, the time to about eight hours still there is a need to shorten the ELISA procedure keeping in mind its enormous application in diagnostics.

The applicants in the present invention have shortened the ELISA procedure by carrying the ELISA procedure on a photoactivated polymer surface at elevated temperature. More particularly, it was carried out on a photoactivated polycarbonate and polystyrene plates at a temperature ranging from 30-70°C. Although microwave- mediated rapid ELISA on an activated surface is available (P. Nahar et al. A rapid method for enzyme- linked immunosorbent assay.

US Patent No. 6,498,016 (2002); PCT Application No. WO 02/14868 A), the technique is highly sensitive and slight change in energy and or time (even in seconds) can spoil the ELISA experiment. The procedure also needs microwave oven or microwave apparatus for carrying out ELISA.

The method according to WO9803038 accelerates known diagnosis methods, as ELISA and (immuno-)staining, by heating samples to be used and controlling the temperature thereof. Preferably this is achieved by microwave radiation. However, some problems are encountered for controlling the temperature within the reaction mixture.

No literature is available for ELISA on a photoactivated surface at elevated temperature (at higher temperature than conventional ELISA usually carried out at 37°C) by conventional heating such as heating in electric oven, incubator, water bath, thermocycler etc.

The main advantage of the present invention is that a slight change in energy or time will not spoil the ELISA experiment in contrast to microwave- mediated ELISA.

The disadvantages of the prior art methods are given in the Table I below

**Table I**

| **Prior arts and their drawbacks** | | |
|---|---|---|
| | **Prior art** | **Drawbacks** |
| 1. | Inert polystyrene surface is activated by nitration, followed by reduction (Aleixo et al. 1985). ELISA carried out by immobilizing antigen onto the amino polystyrene by diazotization reactions. | Antigen immobilization itself is a cumbersome, time-consuming multistep procedure. ELISA by this method is also very time consuming procedure. |
| 2. | Using a preactivated polystyrene microtiter plate by a simple photochemical reaction, enhancement of the speed and sensitivity of an ELISA technique was achieved by Bora et at. (2002). Immobilization of antigen)/antibody onto the photoactivated plates was done in only 45 min with higher binding than that obtained through Adsorption during the same period onto the untreated surface. ELISA was carried out with the solid phase prepared on the photoactivated surface and nearly 1.5 -2-folds higher readings were obtained. | The ELISA procedure is better than the conventional procedure (require about 15 h). Still it requires about 8 h when carried out on the photoactivated polystyrene surface. It is a time consuming procedure specially when used for diagnostic purposes |
| 3. | PCT patent no. WO 02/14868 A1 discloses a method for carrying out enzyme-linked immunosorbent assay by controlled microwave irradiation in less than 10 min. | Procedure needs microwave oven or apparatus for carrying ELISA. This ELISA procedure is very delicate. Slight variation in optimum, condition may hamper the ELISA procedure. |

### Objects of the invention

The main object of the present invention is to provide a heat-mediated ELISA procedure on an activated surface in short time.

Another object of the present invention is to provide a rapid technique, which can be carried out at high temperature using any temperature source.

Another object of the present invention is to provide a rapid technique, which has the potential for automation.

### Summary of the invention

With a view to achieve the objectives and overcoming the disadvantage of known ELISA method, a rapid and efficient method is provided for heat-mediated ELISA (HELISA) which comprises the steps of (i) covalent immobilization of the antigen or antibody on to the activated solid surface by heat, (ii) blocking the free surface with blocking agent by controlled heat, (iii) binding of antibody or antigen by controlled heat (iv) binding of conjugate by controlled heat, (v) adding dye-substrate to the wells and (vi) recording the absorbance value.

Heat-mediated ELISA (HELISA) procedure is carried out on an activated surface in less than 3 h and with the same efficacy as in the conventional ELISA carried out at 37°C, in about 15 h. To overcome the problem of detachment of the solid phase prepared by adsorbtion, the applicants activate the surface of the wells of a PCR plate prior to use.

The activated surface immobilizes the antigen through a covalent bond by -controlled temperature. This covalently immobilized antigen is stable enough to withstand repeated but brief heat exposure, which were needed for performing the subsequent steps of ELISA. Subsequent steps of biomolecule binding in the ELISA procedure are through non covalent binding which are susceptible to heat energy. These problems are overcome by controlling time and temperature in each step.

Novelty of the present invention is that the ELISA is carried out on an activated surface capable of forming covalent linkage with the biomolecule.

In the invented procedure, all steps of ELISA such as antigen binding, blocking, antibody and conjugate binding are carried out by thermal activation in a thermocycler and only enzyme-substrate reaction is performed out side thermocycler at room temperature.

The invented procedure of ELISA is rapid with comparable or even better ELISA value than the conventional method.

Another novelty of the present invention is that the invented ELISA procedure can be fully or partially automated with the use of a specially designed device.

Another novelty of the present invention is that the invented procedure can be used for other immunoassays like radio immunoassay, radio-immunosorbent test, radio allergosorbent test, biotin- avidin /streptavidin immunoassay, immnunoblotting, immunostaining etc. apart from different types of ELISA such as direct ELISA, indirect ELISA, sandwich ELISA and like.

### Detailed description of the invention

The present invention provides a simple approach for enzyme- linked immunosorbent assay technique on an activated polycarbonate or polystyrene surface by heat exposure. Activated surface immobilizes antigen through covalent bonding by thermochemical reaction. This covalently immobilized antigen is stable enough to withstand repeated heat exposure, which are needed for performing the subsequent steps of ELISA. ELISA is a multistep and delicate process where improper condition in any step may hamper the whole result.

In the invented procedure, the first step of ELISA was performed by covalent immobilization of the goat anti-human IgG onto the activated polycarbonate PCR plate by thermal incubation for 1 h in different temperatures. The optimum temperature for antigen immobilization was found as 50°C. In experiments carried out on binding of goat anti-human IgG for the same duration in same incubation temperature on untreated surface showed lower ELISA value (Table 1).

Immobilization of the Goat anti-human IgG onto the activated polycarbonate PCR plate by thermal incubation at 50°C was detectable even in 20 min, which increases with the increase in time of incubation. At 60 min, the antigen binding becomes more or less same as 40 min thermal incubation. Hence, optimum time for antigen immobilization was taken as 40 min (Table 2).

In the second step of HELISA, blocking was carried out with 2% BSA in 1 h at different temperatures in the thermocycler. The optimum temperature for blocking was 40°C. Further, increase in incubation temperature showed non-specific blinding (Table 3)

The optimum time for blocking at 40°C with 2% BSA was found to be 40 min. Further, decrease in incubation time showed inadequate blocking (Table 4).

In the third step of HELISA, antibody binding to the immobilized antigen was carried out in 1 h at different temperatures. Human IgG was found to optimally bind to the solid phase at 50°C (Table 5).

The optimum time for antibody binding to the immobilized antigen at 50°C was found to be 45 min (Table 6).

In the fourth step of HELISA, conjugate binding was carried out in 1 h at different temperatures. Excellent result was obtained at 50°C of incubation (Table 7).

The optimization of conjugate binding time was found in the thermocycler at 50°C by incubating at different duration of time. The best result was obtained in 40 min (Table 8).

In all the experiments, lower ELISA values were obtained when they were carried out on untreated surface.

The applicants have also compared ELISA on activated polycarbonate and polystyrene plates at 37°C. Thus, when ELISA steps such as antigen binding, blocking, antibody binding and antibody-conjugate binding were carried out in 45 min, 1 h, 3 h and 3 h respectively the ELISA values on both the surfaces were found similar (Table 9).

The applicants also compared ELISA values, obtained on activated polycarbonate and polystyrene plates by carrying out ELISA steps at 37°C with HELISA timings such as in 40 min, 40 min, 45 min and 40 min respectively. In both the plates, the results were much lower than conventional ELISA or HELISA (Table 9). It showed that the temperature is responsible for enhanched ELISA.

In another experiment HELISA steps (50°C, 40 min; 40°C, 40 min; 50°C, 45 min and 50°C, 40 min) were carried out on both the activated plates. For PCR polycarbonate plate thermocycler was used for high temperature incubation and for polystyrene microtiter plate, ordinary incubator was used, as normal polystyrene ELISA plates cannot fit into conventional thermocycler. However, incubator temperature for HELISA on polystyrene plate was kept 2°C higher than the thermocycler temperature in each step of ELISA. In incubator a little higher temperature is required as heat transfer is little slow here than in the thermocycler. HELISA can also be carried out on polystyrene plate in an incubator without increasing its temperature but increasing 5 min for each step of ELISA to compensate delay in heat transfer in incubator. In all cases, surfaces without activation showed much lower absorbance value. Results obtained in both these plates were very similar which suggests that heat mediated ELISA (HELISA) can be carried out on activated polystyrene microtiter plate also.

IgE was also detected with same efficacy as IgG by the invented method. The standard curve prepared for quantitative assay of human IgE (Table 10 A) and the amount of IgE present in patient sera (Table 10 B) by HELISA (in 2 h 50 min) and ELISA (in 7 h 50 min) methods showed comparable results (Table 10). Thus, the invented method can reduce assay time to 2 h 50 min from 7 h 50 min without compromising the ELISA value.

The invented HELISA procedure is also very sensitive as seen from Table 11, where it detected IgE in 1/8 dilution of patient sera. Thus, the present invention is rapid, sensitive and simple and can be carried out in any normal laboratory set up.

The total time required for HELISA is less than 3 h. However, time required in each step may vary depending on the biomolecules where excellent results can be obtained by minor modification of reaction conditions that is minor alteration in duration and temperature of incubation. Instead of thermocycler HELISA can also be carried out in an incubator maintaining the temperature of 52°C for antigen binding, antibody binding and conjugate binding, and 42 °C for blocking for a time period of 40, 45, 40 and 40 min respectively.

Accordingly, the present invention provides a rapid method for enzyme-linked immunosorbent assay characterized in using an activated solid support wherein the said method comprises:
(a) providing an activated solid support,
(b) loading a biomolecule selected from an antigen or antibody by dissolving the said biomolecule in a coating buffer into the activated well of the said solid support and heating the said well at a temperature ranging from 40- 80°C for a period ranging from 10 - 70 min followed by washing the well thoroughly with an appropriate washing buffer,
(c) blocking the well having an immobilized biomolecule as obtained from step (b) as above by loading blocking solution into the said well and heating the said well at a temperature ranging from 40- 70 °C for a period ranging from 10- 60 min and washing the said well with an appropriate washing buffer,
(d) loading the corresponding antibody or antigen dissolved in a buffer into the well immobilized with antigen or antibody as obtained from step (c) as above followed by heating the said well at a temperature ranging from 40- 80°C for a period ranging from 10- 70 min followed by washing with washing buffer,
(e) loading an appropriate enzyme- conjugate dissolved in a suitable buffer into the above said well obtained from step (d) and heating the said well at a temperature ranging from 40- 80 °C for a period ranging from 20- 70 min followed by washing with a washing buffer,
(f) adding a substrate-dye-buffer to the above well as obtained from step (e) as above and keeping it for a period ranging from 4 to 10 min in dark followed by addition of stop solution and measuring optical density of the solution by spectrophotometer at a suitable wavelength.

In an embodiment of the present invention the solid support used is selected from the group consisting of materials such as polycarbonate, polystyrene, polypropylene, polyethylene, glass, cellulose, nitrocellulose, silicagel, polyvinyl chloride and like.

In an embodiment of the present invention, the preferred solid supports used are polycarbonate and polystyrene.

In yet another embodiment the solid support is selected from any shape, form and size such as PCR plates, ELISA plate, microwell plate, sheets, test particles such as beads and microspheres, test tubes, test sticks, test strips, well or module.

In an embodiment of the present invention, the activated solid support used for immobilizing biomolecules is selected from any solid support capable of binding ligand molecules particularly by covalent binding.

In yet another embodiment of the present invention, the activated solid support may have active functional group for covalent binding which is selected from halide, aldehyde, acetyl, epoxide, succinamide, isothiocyanate, acylazide and like.

In yet another embodiment of the present invention the active functional group may be present in the support itself or can be introduced by conventional chemical or photochemical or other methods known to prior art.

In yet another embodiment of the present invention the functional group is introduced on to the solid support by photochemical reaction using a photoactive compound which is selected from 4-azido-1-fluoro-2-nitrobenzene (1-fluoro-2-nitro-4-azidobenzene), N-hydroxysulfo-succinimidyl 4-azidobenzoate, N-hydroxysulfo-succinimidyl 4-azidosalicyclic acid, quinone or its derivatives, and like.

In yet another embodiment of the present invention, polycarbonate and polystyrene surface are activated by coating 4-azido-1-fluoro-2-nitrobenzene and exposing the coated support to UV radiation at 365 nm.

In yet another embodiment of the present invention, the light source for photochemical reaction is selected from UV lamp, laser beam, bright sunlight or like.

In yet another embodiment of the present invention, time for photoreaction for activation of solid support is selected from 10 seconds to 10 hours.

In yet another embodiment of the invention, incubation is performed in an apparatus selected from laboratory polymerase chain reaction (PCR) thermocycler, specially designed thermocycler, incubator, water bath or any apparatus or chamber in which heat is generated and like.

In yet another preferred embodiment of the invention, the first step of ELISA, is carried out by covalent binding of antigen or antibody onto the activated plate by heating at a temperature ranging from 40-80°C for a period ranging from 10-70 min.

In yet another preferred embodiment of the invention the second step of ELISA, that is the blocking step is carried out by heating at a temperature ranging from 40-70°C for a period ranging from 10-60 min.

In yet another preferred embodiment of the invention the third step of ELISA, that is corresponding antibody or antigen binding is carried out by heating at a temperature ranging from 40-80°C for a period ranging from 10-70 min.

In yet another preferred embodiment of the invention, the fourth step of ELISA that is enzyme-conjugate binding is carried out by heating at a temperature ranging from 40-80°C for a period ranging from 10-70 min.

In yet another preferred embodiment of the invention the total time for antigen binding, blocking, antibody binding and conjugate binding is ranging from 2-4 h wherein the total time for conventional ELISA method usually ranges from 8 h to 24 h.

In another embodiment to the present invention, the antigen can be dissolved in a coating buffer such as carbonate buffer, phosphate buffer and like of suitable composition having a pH, in the range of from 6.5 to 11 with molarity ranging from 0.005 M to 0.1 M and should be compatible with the antigen.

In yet another preferred embodiment of the invention, washing buffer used is a mixture of phosphate buffer having a pH, in the range of from 6.5 to 11, with molarity ranging from 0.005 M to 0.1 M and tween 20 in the range of between 0.05% to 3%.

In yet another preferred embodiment of the invention, blocking reagent is selected from bovine serum albumin, skimmed milk powder, and gelatin and like.

In another embodiment to the present invention biomolecule is selected from antigen or antibody. Antigen may be any biomolecule, microorganism, etc that elicits or has the potential to elicit an immune response.

In another embodiment to the present invention conjugare is selected from biomolecule having antibody or antigen conjugated with an enzyme selected from horseradish peroxidase or alkaline phosphatase. In yet another preferred embodiment of this invention, enzyme may be replaced by a label selected from chromophore, fluorophore and like which facilitates its assay. In yet another preferred embodiment of this invention, the invented procedure can be used for other immunoassays like radio immunoassay, radio-immunosorbent test, radio allergosorbent test, biotin- avidin/streptavidin immunoassay, immnunoblotting, immunostaining etc. apart from different types of ELISA such as direct ELISA, indirect ELISA, sandwich ELISA and like.

In yet another preferred embodiment of this invention, the invented procedure can be automated or semiautomated. An apparatus may be made to fit microtiter ELISA plates for heat-mediated enzyme linked immunosorbent assay (HELISA) comprising a reaction chamber consisting of rapid heating system for temperature ranging from 40-70°C and a cooling system such as exhaust fan for carrying out the steps of antigen binding, blocking, antibody binding and antibody enzyme conjugate binding.

In the invented procedure, inert solid surface such as polycarbonate PCR plate (usally used for polymerase chain reaction) and 96-well polystyrene microtiter plate were activated by a photochemical reaction carried out in dry condition using FNAB. The solid support was activated by exposing the FNAB coated support to UV radiation or bright sunlight. This activated support was used for heat- mediated ELISA (HELISA) and for control ELISA to detect antibodies, to example goat anti-human IgG and goat anti-human IgE.

Thermal reaction was performed inside a thermocycler (Perkin Elmer, Gene Amp PCR System 2400) or in a BOD incubator. Carbonate buffer, pH 9.6,0.1 M was used as coating buffer.

Phosphate buffer saline (PBS), pH 7.2,0.01 M was used as antibody dilution buffer.

Phosphate buffer saline (PBS), pH 7.2, 0.01 M together with 0.1% tween 20 was used as washing buffer in all the experiments unless mentioned otherwise. Blocking solution was made by dissolving 2% BSA in 0.01 M PBS, pH 7.2. Substrate solution was prepared by adding 0.067% of o-phenylenediamine and 0.043% of H₂O₂ in 0.1 M phosphate citrate buffer, pH 4.5). Normal goat sera were taken as negative control sera (-ve sera). 100 µl of diluted (1:300) -ve sera was used for each well.

Horse radish peroxidase conjugated anti-rabbit IgG was purchased from Sigma as lyophilized powder.

After reconstitution, the optimum dilution was found to be 1: 5000 as determined by checkerboard titration, which was used in the experiments.

In all the ELISA experiments, absorbance values are expressed as mean of three replicates.

ELISA is a 5-step procedure, namely antigen binding, blocking, antibody binding, conjugate binding and color development. Each step of HELISA was optimized by carrying out the subsequent steps by conventional ELISA procedure. Conventional ELISA was carried out by coating the wells with antigen overnight at 4°C, (45 min at 37°C for activated wells), blocking in 1 h at 37°C followed by antibody and conjugate binding at 37°C for 3 h each and color development that is enzyme-substrate reaction at room temperature for 5 min followed by reading absorbance.

| **The present invention and its advantages** | | |
|---|---|---|
| | **Present invention** | **Advantages** |
| 1. | Present invention provides a method for Heat-mediated ELISA (HELISA) procedure by doing it on an activated polymer surface capable of forming a covalent bond with antigen or antibody. | 1. Present invention provides an ELISA procedure, which takes around 3h, less than required in conventional procedure (usually 10-15 h). 2. The procedure even takes less than the recently published procedure of ELISA (about 8 h) (Bora et al. 2002) on the photoactivated surface 3. The invented procedure is sensitive and requires minute quantities of precious antigen or antibody. 4. The invented procedure is simple and does not require any additional expertise, reagent, or equipment. It can be carried out on common laboratory apparatus such as thermocycler, incubator or even on water bath. 5. The procedure can be easily automated. 6. A small deviation in the incubation conditions from the optimum conditions does not effect the ELISA value significantly in the present invention. |

This invention is further explained with the help of the following examples and should not be construed to limit the scope of the invention.

### Example 1- Activation of polycarbonate and polystyrene surfaces

Wells of a PCR plate (polycarbonate plate, Greiner, Germany) were loaded with 6.0 µmol of 1-flouro-2-nitro-4-azidobenzene (FNAB), dissolved in 50 µl of methanol per well and dried completely in the dark. FNAB coated wells were then irradiated for 7.5 min with UV irradiation of 365 nm wavelength in a UV Stratalinker 2400 (Stratagene®, USA) or kept under bright sunlight for 15 min. Wells of polystyrene microtiter plate were activated similarly using 10 µmol of FNAB, dissolved in 50 µl methanol dried and irradiated for 10 min at 365 nm or sunlight for 15 min.

The wells were then washed several times with methanol to remove the unbound linker and dried at room temperature. These activated wells were used for immobilization of antigen or antibody in the invented procedure.

### Example 2: Determination of goat anti-human IgG concentration for the preparation of solid phase on activated polycarbonate surface

A double dilution series (2000 - 0.061 g/ml) of goat anti-human IgG (dissolved 0.1 M carbonate/ bicarbonate buffer, pH 9.6) was loaded in triplicate wells (90 µl/well for each dilution) of an activated and untreated polycarbonate plates. The plates were then incubated at 50°C for 1 h in a thermocycler. The plates were then washed for six times with washing buffer (0.05% Tween 20 with 0.01 M PBS) and blocking step was done with with 2% BSA solution (100 µl/well) at 37°C in 1 h. After washing, each well was loaded with 90 µl of a 250 ng/ml of a human IgG solution (dissolved in 0.01 M PBS, pH 7.4) and the plates were incubated at 37° for 3 h. The plates were again washed for six times and each well was loaded with 90 µl of a 1/5000 (v/v), goat anti-human IgG-peroxidase conjugate solution (dissolved in 0.01 M PBS, pH 7.4) and incubated at 37°C for 3 h. After washing, 90 µl of substrate-dye solution (0.4 mg o-phenylenediamine hydrochloride and 2 µl H₂O₂ in 10 ml of 0.2 M citrate buffer, pH 5) was loaded into each well. Colour development was stopped after 5 min by adding 10 µl of 5% H₂ SO₄. The solutions were transferred into wells of a polystyrene microtitre plate and absorbance was recorded at 490 nm.

### Example 3: Optimization of temperature for immobilization of goat anti-human IgG on the activated and untreated polycarbonate plates (Table 1)

Triplicate wells of eight activated polycarbonate plates, were loaded with goat anti-human IgG solution (90 µl/well of a 250 ng/ml solution) and incubated for 60 min at 35, 40, 45, 50, 55, 60, 65 and 70°C respectively in a thermocycler. Goat anti-human IgG was also similarly immobilized in untreated polycarbonate plates. The remaining steps of ELISA were then carried out with these solid phases by conventional ELISA method as described in example 2.

### Example 4: Optimization of goat anti-human IgG incubation time for the preparation of solid phase (Table 2)

Triplicate wells of six activated and six untreated polycarbonate plates were loaded with 90 µl/well of goat anti-human IgG (250 ng/ml) and plates were incubated at 50°C for 10 20, 30, 40, 50 and 60 min respectively in a thermocycler. Remaining steps of ELISA were carried out with these solid phases by conventional ELISA method described in example 2.

### Example 5: Studies on the incubation temperature of blocking step (Table 3)

Goat-anti human IgG was immobilized as described above on the triplicate wells of six activated plates at 50°C in 40 min incubation. After washing, the wells were loaded with 100 µl of a 2% (w/v) BSA solution. The plates were then incubated for 1 h at 40, 45, 50, 55, 60 and 65°C respectively. Incubation of human IgG and second antibody-enzyme conjugate were done at 37°C for 3 h each. Control experiments with untreated plates were carried for each corresponding temperature similarly.

### Example 6: Optimization of incubation time for blocking step (Table 4)

Goat-anti human IgG was immobilized as described in example 5 on the triplicate wells of six activated plates. After washing, the wells were loaded with 100 µl of a 2% (w/v) BSA solution. The plates were then incubated at 40°C for 10, 20, 30, 40, 50 and 60 min respectively. Human IgG and second antibody-enzyme conjugate were incubated at 37°C for 3 h each. Control experiments with untreated plates were carried for each corresponding time.

### Example 7: Optimum dose of human IgG required for the assay

The solid phases were prepared by immobilizing goat anti-human IgG (250 ng/ml) in activated and untreated wells of a polycarbonate plate at 50°C for 40 min. Washing and blocking (40°C, 40 min) was done as described previously. The wells were then loaded with a two fold dilution series (1000-0.122 ng/ml) of a human IgG solution and incubated for 3 h at 37°C. The remaining steps of ELISA was carried as previously described.

### Example 8: Determination of optimum temperature for the binding of human IgG with the solid phase (Table 5)

The solid phase was prepared on an activated polycarbonate plate as described in example 5. Blocking was done under optimal conditions (40°C, 40 min) as above. After washing, 90 µl of a human IgG (from a solution of 125 ng/ml) was incubated with the solid phase for 60 min at 40°C. The plate was washed and a 90 µl of 1/5000 dilution of goat anti-human IgG peroxidase conjugate was incubated for 3 h at 37°C. The absorbance was recorded as described. Similarly, five more experiments were done by incrementing the temperature of incubation of human IgG by 5°C up to 65°C. Control experiments were carried out with untreated polycarbonate plates for corresponding temperature.

### Example 9: Binding of human IgG onto the solid phase in different incubation time (Table 6)

90 µl of human IgG solution (from a solution of 125 ng/ml) was incubated at 50°C for 15 min with solid phase prepared as in example 8. The washed wells were then incubated with a 1/5000 dilution of goat anti-human IgG- peroxidase at 37°C for 3 h and assayed as previously described. Four more experiments were carried out by incubating human IgG for 30, 45, 60 and 90 min respectively. Control experiments were carried out with untreated polycarbonate surface under similar conditions.

### Example 10: Optimum temperature for binding of goat anti-human IgG peroxidase conjugate with human IgG bound to solid phase (Table 7)

Preparation of solid phase and blocking were carried out as in example 8. Incubation of human IgG was carried out at 50°C for 4.5 min.as above. 1/5000 v/v dilution of goat anti-human IgG-peroxidase (90 µl/well) was then incubated for 60 min at 40°C. After washing, absorbance was recorded as above. Five more experiments were done similarly at different incubation temperatures (45, 50, 55, 60 and 65°C) of second antibody-enzyme conjugate. Control experiments were carried out with untreated surface under similar conditions.

### Example 11: Optimization of incubation time for binding of goat anti-human IgG peroxidase conjugate to solid phase-bound human IgG (Table 8)

Immobilization of goat anti-human IgG, blocking and human IgG were incubated as in example 10. Second antibody-enzyme conjugate (1/5000, v/v) was incubated at 50°C for 10 min and assayed. Five more experiments were carried out by incubating the conjugate at 20, 30, 40, 50 and 60 min respectively. Control experiments were carried out in corresponding time with untreated surface.

### Example 12: Comparative studies of HELISA and conventional ELISA on polycarbonate and polystyrene surfaces for the detection of human IgG (Table 9)

In polycarbonate (PC 1) and polystyrene (PS 1) plates, ELISAs were performed by immobilizing goat anti-human IgG in 45 min at 37°C, blocking in 1 h at 37°C, human IgG binding in 3 h at 37°C and conjugate binding in 3 h at 37°C. In plate PC 2 and PS 2 ELISAs were performed by immobilizing goat anti-human IgG in 40 min, blocking in 40 min, binding human IgG in 45 min and conjugate binding in 40 min at 37°C. In plate PC 3 HELISA was performed by immobilizing goat anti-human IgG in 40 min at 50°C, blocking in 40 min at 40°C, binding human IgG in 45 min at 50°C and conjugate binding in 40 min at 50°C. HELISA on PS 3 plate were carried out in an incubator similarly as in PC 3 except that the temperature of the incubator in each step was 2°C higher then PC 3 experiment carried out in a thermocycler. All these experiments were carried out with activated and untreated plates.

### Example 13: Quantitative determination of IgE in patient sera by HELISA and conventional ELISA procedures (Table 10)

### A. Preparation of standard curves by HELISA and conventional ELISA procedures (Table 10 A)

In HELISA procedure, goat anti-human IgE (0.75 µl of a 1 µg/ml solution) was immobilized into activated polycarbonate wells in 40 min at 50°C. The plate was washed for six times with washing buffer (50 mM tris, 0.14 NaCI, pH 8.0 and 0.05% tween 20). Blocking was done with 2% BSA by incubating the plate at 40°C for 40 min. The wells were then loaded with 0.75 µl of IgE standards (Bethyl Laboratories Inc., USA) in a double dilution series (1000, 500,250,125, 62.5, 31.25 and 15.34 ng/ml) and incubated for 45 min at 50°C. After washing, the wells were loaded with 0.75 µl of goat anti-human IgE peroxidase solution and incubated for 40 min at 50°C. Plate was again washed and assayed for enzyme.

The conventional ELISA was carried out by immobilizing the capture antibody (goat anti-human IgE) on activated surface by incubating 45 min. at 37°C or overnight at 4°C. Blocking was done in 1 h at 37°C. The standard IgE, and conjugate were incubated for 3 h each at 37°C.

### B. Determination of IgE in patient's sera (Table 10 B)

HELISA and conventional ELISA were carried out similarly except that 1/4 (v/v, diluted) patient's sera were used instead of IgE standard for determining IgE in patient's sera.

### Example 14: Sensitivity of the solid phase in detecting IgE in patients sera (Table 11)

The capture antibody was immobized in 40 min at 50°C in both untreated and activated polycarbonate wells. After blocking in 40 min at 40°C, the wells were loaded with human blood sera in different dilutions (1/4, 1/8, 1/16 and 1/32) and incubated for 45 min at 50°C. Conjugate incubation was at 50°C in 40 min. Colour development was at ambient temperature for 5 min. Absorbance recorded as described earliar.

### Table 1. Detection of human IgG in by carrying out first step by HELISA and remaining steps by ELISA procedures.

HELISA: Step-1. Immobilization of anti-human IgG on activated and untreated wells by thermal incubation for 1 h in different temperatures as in the table.

ELISA: (Step 2 to 5- Conventional procedure) blocking- 1 h, 37°C; antibody incubation-37°C, 3 h and conjugate binding- 37°C, 3 h. Color development- 5 min, room temperature.

| **Tempera ture °C** | **Activated plate** | | | | **Untreated plate** | | | |
|---|---|---|---|---|---|---|---|---|
| | **+ ve sera** | **±sd** | **- ve sera** | **±sd** | **+ ve sera** | **±sd** | **- ve sera** | **±sd** |
| 35 | 0.442 | 0.011 | 0.117 | 0.005 | 0.350 | 0.007 | 0.105 | 0.006 |
| 40 | 0.510 | 0.006 | 0.115 | 0.011 | 0.348 | 0.016 | 0.114 | 0.006 |
| 45 | 0.633 | 0.010 | 0.104 | 0.024 | 0.350 | 0.009 | 0.104 | 0.012 |
| 50 | 0.675 | 0.014 | 0.112 | 0.019 | 0.333 | 0.012 | 0.101 | 0.018 |
| 55 | 0.642 | 0.005 | 0.099 | 0.025 | 0.305 | 0.006 | 0.097 | 0.023 |
| 60 | 0.560 | 0.008 | 0.115 | 0.003 | 0.279 | 0.008 | 0.095 | 0.022 |
| 65 | 0.411 | 0.006 | 0.094 | 0.021 | 0.261 | 0.007 | 0.095 | 0.021 |
| 70 | 0.343 | 0.008 | 0.076 | 0.009 | 0.209 | 0.004 | 0.068 | 0.007 |

### Table 2. Detection of human IgG by carrying out first step by HELISA and remaining steps by ELISA procedures.

HELISA: Step-1. Immobilization of anti-human IgG by thermal incubation at 50°C in different time as in the table.

ELISA: (Step 2 to 5- Conventional procedure) blocking- 1 h, 37°C; antibody incubation-37°C, 3 h and conjugate binding- 37°C, 3 h. Color development- 5 min, room temperature

| **Time (min)** | **Activated plate** | | | | **Untreated plate** | | | |
|---|---|---|---|---|---|---|---|---|
| | **+ ve** sera | **±sd** | **- ve** sera | **±sd** | **+ ve** sera | **±sd** | **- ve** sera | **±sd** |
| 10 | 0.226 | 0.009 | 0.089 | 0.011 | 0.172 | 0.003 | 0.102 | 0.005 |
| 20 | 0.295 | 0.020 | 0.106 | 0.011 | 0.197 | 0.009 | 0.112 | 0.005 |
| 30 | 0.478 | 0.006 | 0.111 | 0.01 | 0.267 | 0.008 | 0.101 | 0.013 |
| 40 | 0.623 | 0.01 | 0.108 | 0.004 | 0.319 | 0.004 | 0.108 | 0.014 |
| 50 | 0.624 | 0.013 | 0.118 | 0.004 | 0.346 | 0.013 | 0.098 | 0.008 |
| 60 | 0.622 | 0.006 | 0.123 | 0.004 | 0.378 | 0.010 | 0.114 | 0.002 |

### Table 3: Detection of human IgG by carrying out first two steps by HELISA and remaining steps by ELISA procedure.

HELISA: Step-1. Anti-human IgG binding- 50°C, 40 min.

Step-2. Blocking- variable temperature as in the table, 1 h.

ELISA: (Step 3 to 5- Conventional procedure) antibody incubation- 37°C, 3 h and conjugate binding- 37°C, 3 h. Color development- 5 min, room temperature

| **Tempera ture °C** | **Activated plate** | | | | **Untreated plate** | | | |
|---|---|---|---|---|---|---|---|---|
| | **+ ve sera** | **±sd** | **- ve sera** | **±sd** | **+ ve sera** | **±sd** | **- ve sera** | **±sd** |
| 40 | 0.576 | 0.009 | 0.076 | 0.003 | 0.324 | 0.003 | 0.069 | 0.003 |
| 45 | 0.563 | 0.010 | 0.075 | 0.001 | 0.318 | 0.005 | 0.081 | 0.014 |
| 50 | 0.549 | 0.008 | 0.172 | 0.006 | 0.319 | 0.006 | 0.123 | 0.010 |
| 55 | 0.616 | 0.006 | 0.183 | 0.005 | 0.336 | 0.009 | 0.135 | 0.010 |
| 60 | 0.618 | 0.006 | 0.200 | 0.008 | 0.353 | 0.005 | 0.173 | 0.005 |
| 65 | 0.633 | 0.005 | 0.208 | 0.005 | 0.358 | 0.005 | 0.194 | 0.008 |

### Table 4: Detection of human IgG by carrying out first two steps by HELISA and remaining steps by ELISA procedures.

HELISA: Step-1. Anti-human IgG binding- 50°C, 40 min.

Step-2. Blocking- variable time as in the table, 40°C.

ELISA: (Step 3 to 5- Conventional procedure) antibody incubation- 37°C, 3 h and conjugate binding- 37°C, 3 h. Color development- 5 min, room temperature

| **Time (min)** | **Activated plate** | | | | **Untreated plate** | | | |
|---|---|---|---|---|---|---|---|---|
| | **+ ve sera** | **±sd** | **- ve sera** | **±sd** | **+ ve sera** | **±sd** | **- ve sera** | **±sd** |
| 10 | 0.66 | 0.006 | 0.228 | 0.004 | 0.322 | 0.007 | 0.12 | 0.008 |
| 20 | 0.655 | 0.007 | 0.149 | 0.007 | 0.302 | 0.007 | 0.113 | 0.007 |
| 30 | 0.620 | 0.005 | 0.097 | 0.013 | 0.278 | 0.007 | 0.079 | 0.006 |
| 40 | 0.608 | 0.006 | 0.075 | 0.011 | 0.28 | 0.004 | 0.057 | 0.01 |
| 50 | 0.613 | 0.002 | 0.067 | 0.013 | 0.274 | 0.005 | 0.048 | 0.006 |
| 60 | 0.609 | 0.003 | 0.069 | 0.008 | 0.281 | 0.008 | 0.048 | 0.005 |

### Table 5: Detection of human IgG by carrying out first three steps by HELISA and remaining steps by ELISA procedures.

HELISA: Step-1. Anti-human IgG binding- 50°C, 40 min.

Step-2. Blocking- 40°C, 40 min.

Step-3. Human IgG binding-variable temperature as in the table, 1h.

ELISA: (Step 4 to 5- Conventional procedure) conjugate binding- 37°C, 3 h. Color development- 5 min, room temperature

| **Temper ature °C** | **Activated plate** | | | | **Untreated plate** | | | |
|---|---|---|---|---|---|---|---|---|
| | **+ ve sera** | **±sd** | **- ve sera** | **±sd** | **+ ve sera** | **±sd** | **- ve sera** | **±sd** |
| 40 | 0.419 | 0.004 | 0.063 | 0.015 | 0.318 | 0.005 | 0.066 | 0.005 |
| 45 | 0.531 | 0.013 | 0.085 | 0.003 | 0.309 | 0.003 | 0.073 | 0.012 |
| 50 | 0.613 | 0.006 | 0.105 | 0.005 | 0.281 | 0.006 | 0.090 | 0.009 |
| 55 | 0.607 | 0.006 | 0.078 | 0.017 | 0.271 | 0.008 | 0.086 | 0.004 |
| 60 | 0.576 | 0.009 | 0.098 | 0.008 | 0.268 | 0.006 | 0.078 | 0.012 |
| 65 | 0.534 | 0.006 | 0.080 | 0.004 | 0.264 | 0.003 | 0.081 | 0.011 |

### Table 6: Detection of human IgG by carrying out first three steps by HELISA and remaining steps by ELISA procedure.

HELISA: Step-1. Anti-human IgG binding- 50°C, 40 min.

Step-2. Blocking- 40°C, 40 min.

Step-3. Human IgG binding-variable time as in the table, 50°C.

ELISA: (Step 4 to 5- Conventional procedure) conjugate binding- 37°C, 3 h. Color development- 5 min, room temperature

| **Time (min)** | **Activated plate** | | | | **Untreated plate** | | | |
|---|---|---|---|---|---|---|---|---|
| | **+ ve sera** | **±sd** | **- ve sera** | **±sd** | **+ ve sera** | **±sd** | **- ve sera** | **±sd** |
| 15 | 0.234 | 0.008 | 0.088 | 0.006 | 0.164 | 0.009 | 0.068 | 0.011 |
| 30 | 0.423 | 0.006 | 0.104 | 0.003 | 0.236 | 0.009 | 0.070 | 0.011 |
| 45 | 0.620 | 0.007 | 0.091 | 0.011 | 0.281 | 0.008 | 0.071 | 0.027 |
| 60 | 0.623 | 0.015 | 0.081 | 0.006 | 0.309 | 0.008 | 0.080 | 0.003 |
| 90 | 0.615 | 0.012 | 0.098 | 0.008 | 0.337 | 0.006 | 0.075 | 0.008 |

### Table 7: Detection of human IgG by carrying out first four steps by HELISA and last step by conventional procedure.

HELISA: Step-1. Anti-human IgG binding- 50°C, 40 min.

Step-2. Blocking- 40°C, 40 min.

Step-3. Human IgG binding- 50°C, 45 min.

Step-4. Conjugate binding- variable temperature as in the table, 60 min.

ELISA; (Step-5) Color development- 5 min at room temperature.

| **Tempera ture °C** | **Activated plate** | | | | **Untreated plate** | | | |
|---|---|---|---|---|---|---|---|---|
| | **+ ve sera** | **±sd** | **- ve sera** | **±sd** | **+ ve sera** | **±sd** | **- ve sera** | **±sd** |
| 40 | 0.407 | 0.012 | 0.108 | 0.012 | 0.335 | 0.007 | 0.073 | 0.008 |
| 45 | 0.585 | 0.010 | 0.081 | 0.010 | 0.331 | 0.008 | 0.056 | 0.011 |
| 50 | 0.614 | 0.010 | 0.084 | 0.005 | 0.299 | 0.010 | 0.067 | 0.005 |
| 55 | 0.607 | 0.005 | 0.088 | 0.009 | 0.282 | 0.007 | 0.073 | 0.003 |
| 60 | 0.565 | 0.012 | 0.077 | 0.004 | 0.250 | 0.006 | 0.077 | 0.016 |
| 65 | 0.524 | 0.009 | 0.057 | 0.011 | 0.238 | 0.004 | 0.055 | 0.011 |

### Table 8: Detection of human IgG by carrying out first four steps by HELISA and last step by conventional procedure.

HELISA: Step-1. Anti-human IgG binding- 50°C, 40 min.

Step-2. Blocking- 40°C, 40 min.

Step-3. Human IgG binding-50°C, 45 min.

Step-4. Conjugate binding- variable time as in the table, 50°C.

ELISA: (Step - 5) Color development- 5 min at room temperature.

| **Time (min)** | **Activated plate** | | | | **Untreated plate** | | | |
|---|---|---|---|---|---|---|---|---|
| | **+ ve sera** | **±sd** | **- ve sera** | **±sd** | **+ ve sera** | **±sd** | **- ve sera** | **±sd** |
| 10 | 0.302 | 0.013 | 0.05 | 0.007 | 0.183 | 0.006 | 0.063 | 0.01 |
| 20 | 0.403 | 0.012 | 0.055 | 0.013 | 0.223 | 0.004 | 0.056 | 0.009 |
| 30 | 0.543 | 0.011 | 0.07 | 0.006 | 0.247 | 0.008 | 0.057 | 0.005 |
| 40 | 0.618 | 0.008 | 0.083 | 0.009 | 0.281 | 0.004 | 0.074 | 0.008 |
| 50 | 0.615 | 0.007 | 0.078 | 0.012 | 0.307 | 0.005 | 0.081 | 0.008 |
| 60 | 0.623 | 0.008 | 0.111 | 0.007 | 0.321 | 0.004 | 0.091 | 0.005 |

### Table 9: Detection of human IgG: Comparison of HELISA and ELISA in FNAB activated and untreated polycarbonate (PC) and polystyrene (PS) plates.

Plates PC 1 and PS 1: Step-1. Goat anti-human IgG binding- 37°C, 45 min. Step-2. Blocking-37°C, 1 h. Step-3. Human IgG binding- 37°C, 3 h. Step-4. Conjugate binding- 37°C, 3 h. Step - 5. Color development- 5 min at room temperature.

Plates PC 2 and PS 2: Step-1. Goat anti-human IgG binding- 37°C, 40 min. Step-2. Blocking-37°C, 40 min. Step-3. Human IgG binding- 37°C, 45 min. Step-4. Conjugate binding- 37°C, 40 min. Step - 5. Color development- 5 min at room temperature.

Pates PC 3 and PS 3*: Step-1. Goat anti-human IgG binding- 50°C (52°C^{‡}), 40 min. Step-2. Blocking- 40°C (42°C^{‡}), 40 min. Step-3. Human IgG binding- 50°C (52°C^{‡}), 45 min. Step-4. Conjugate binding- 50°C (52°C^{‡}), 40 min. Step - 5. Color development- 5 min at room temperature.

| **Treatm ents** | **Activated plate** | | | | **Untreated plate** | | | |
|---|---|---|---|---|---|---|---|---|
| | **+ ve sera** | **±sd** | **- ve sera** | **±sd** | **+ ve sera** | **±sd** | **- ve sera** | **±sd** |
| PC1 | 0.552 | 0.017 | 0.082 | 0.01 | 0.276 | 0.003 | 0.083 | 0.003 |
| PS1 | 0.545 | 0.007 | 0.099 | 0.014 | 0.261 | 0.008 | 0.079 | 0.004 |
| PC2 | 0.376 | 0.007 | 0.073 | 0.006 | 0.258 | 0.005 | 0.077 | 0.005 |
| PS2 | 0.359 | 0.004 | 0.071 | 0.007 | 0.219 | 0.006 | 0.067 | 0.022 |
| PC3 | 0.626 | 0.009 | 0.113 | 0.005 | 0.280 | 0.009 | 0.074 | 0.007 |
| PS3 | 0.615 | 0.005 | 0.071 | 0.007 | 0.246 | 0.005 | 0.063 | 0.02 |

| | | | | | | | | |
|---|---|---|---|---|---|---|---|---|
| * PS-3^{:} HELISA was carried out in BOD incubator ^{‡} Temperature for PS-3 | | | | | | | | |

### Table 10: Quantitative assay of human IgE antibodies: Comparision of HELISA and ELISA procedures on activated polycarbonate plates.

HELISA: Step-1. Goat anti-human IgE binding- 50°C, 40 min. Step-2. Blocking- 40°C, 40 min. Step-3. Standard (Table 10 A) and patient's (Table 10 B) sera incubation - 50°C, 45 min. Step-4. Conjugate binding- 50°C, 40 min. Step - 5. Color development- 5 min at room temperature.

ELISA: Step-1. Goat anti-human IgG binding- 37°C, 45 min.; Step-2. Blocking- 37°C, 1h; Step-3. Standard (Table 10 A) and patient's sera ((Table 10 B) incubation - 37°C, 3 h; Step-4. Conjugate binding- 37°C, 3 h. Step - 5. Color development- 5 min at room temperature.

**Table 10 A**

| **Standard IgE (ng/ml)** | **HELISA** | | **ELISA** | |
|---|---|---|---|---|
| | **Mean** | **±sd** | **Mean** | **±sd** |
| 15.625 | 0.067 | 0.006 | 0.041 | 0.004 |
| 31.25 | 0.149 | 0.007 | 0.125 | 0.003 |
| 62.5 | 0.266 | 0.009 | 0.247 | 0.006 |
| 125 | 0.357 | 0.009 | 0.362 | 0.005 |
| 250 | 0.444 | 0.010 | 0.460 | 0.005 |
| 500 | 0.535 | 0.007 | 0.549 | 0.006 |
| 1000 | 0.675 | 0.008 | 0.653 | 0.010 |

**Table 10 B**

| **Sample No.** | **HELISA** | | **ELISA** | |
|---|---|---|---|---|
| | **Mean** | **±sd** | **Mean** | **±sd** |
| 1 | 0.376 | 0.005 | 0.369 | 0.004 |
| 2 | 0.340 | 0.007 | 0.342 | 0.005 |

### Table 11: Comparison of sensitivity of the activated and untreated polycarbonate plate towards the detection of IgE in diluted sera.

Step-1. Goat anti-human IgE binding- 50°C, 40 min. Step-2. Blocking- 40°C, 40 min. Step-3: Patient's sera incubation- 50°C, 45 min. Step-4. Conjugate binding- 50°C, 40 min. Step - 5. Color development- 5 min at room temperature.

| **Sera dilution** | **Activated plate** | | **Untreated plate** | |
|---|---|---|---|---|
| | **+ ve sera** | **±sd** | **+ ve sera** | **±sd** |
| ¼ | 0.377 | 0.007 | 0.223 | 0.005 |
| 1/8 | 0.230 | 0.024 | 0.150 | 0.006 |
| 1/16 | 0.094 | 0.007 | 0.075 | 0.012 |
| 1/32 | 0.078 | 0.012 | 0.075 | 0.003 |

### Advantages

Conventional methods of ELISA usually take 8-15 h for completion, which is the major drawback for a procedure used worldwide in different fields apart from clinical diagnostics. In case of medical urgency, precious time is lost in diagnosis before the patient could be given medication. Therefore, a rapid ELISA procedure invented herein (HELISA) will be beneficial and useful for diagnosis of diseases, biomedical research and other related fields. Main advantages of the invented ELISA procedure are:
1. The invented procedure is rapid than the conventional method of ELISA.
2. The total time required in the invented method is less than 3 h. Thus, it obviates the time consuming procedure.
3. The invented procedure is sensitive and requires minute quantities of precious antigen or antibody.
4. The procedure is simple and does not require any additional expertise or reagent to do it.
5. The invented procedure is cost effective and does not require any additional equipment except a heating apparatus, which is common in most of the laboratories.
6. The invented procedure is reproducible which is an important criterion for ELISA.
7. The procedure gives minimal or negligible non-specific binding.
8. The procedure has the potential for automation, which can minimize human error.

### References.

1. Douillard, J.Y. and Hoffman, T. (1983) Methods in Enzymology 92, 168-74.
2. Van Emon, J.M. and Lopez-Avila, V. (1992) Analytical Chemistry 64, 79 A-88A.
3. Ghassempour, A. Mohammadkhah, A., Najafi, F. and Rajabdeh, M. (2002) Anal. Sci. 8, 779-83.
4. Salgame, P., Varadhachary, A.S, Primiano, L.L., Finke, J.E., Muller, S. and Monestier, M. (1996) Nucleic Acids Res. 25, 680-1.
5. Kricka, L.J., Carter, T.J., Burt, S.M., Kennedy, J.H., Holder, R.L. and Halliday, M.I., 1980. Clin. Chem. 26, 741-4.
6. Hermann, J.E. and Collins, M.F. (1976) J. Immunol. Methods 10, 363-6.
7. Engvall, E. and Perlmann, P. (1971) Immunochemistry 8, 871-4.
8. Kemeny, D.M., 1997. In: Johnstone, A.P., Turner, M.W. (Eds.), Immunochemistry I: A Practical Approach. IRL Press at Oxford Univ. Press, New York, and pp.-147-175.
9. Deshpande, S.S. (1996) Enzyme Immunoassays from Concept to Product Development. Chapman & Hall, New York.
10. Douglas, A.S. and Monteith, C.A. (1994) Clin. Chem. 40, 1833-37.
11. Larsson, P.H., Johansson, S.G.O., Hult, A. and Gothe, S. (1987) J. Immunol. Methods 98, 129-35.
12. Aleixo, J.A.G., Swaminathan, B., Minnich, S.A. and Wallshein, V.A. (1985) J. Immunoassay 6, 391-401.
13. Bora, U., Chugh, L. and Nahar, P. (2002). J. Immunol. Methods 268, 171-8.
14. Nahar, P., Wali, N.M. and Gandhi, R.P. (2001) Anal. Biochem. 294, 148-53.

### Other References

### Patent Documents

1. P.Nahar. A method for photochemical activation of polymer surface and immobilization of biomolecules onto the activated surface. **US patent filed June, 2002, NF 304/02**
2. P. Nahar, U. Bora and G.L. Sharma. (2002) A rapid method for enzyme- linked immunosorbent assay. US patent no. 6,498,016
3. P. Nahar, U. Bora and G.L. Sharma. A rapid method for enzyme- linked immunosorbent assay. PCT patent no. WO 02/14868 A1

## Claims

1. A rapid method for enzyme-linked immunosorbent assay **characterised in** using an activated solid support wherein the said method comprises:
a. providing an activated solid support with at least one activated well,
b. loading a biomolecule selected from an antigen or antibody by dissolving the said biomolecule in a coating buffer and into the activated well of the solid support and heating the said well at a temperature ranging from 40-80 °C for a period ranging from 10-70 min followed by washing the well thoroughly with an appropriate washing buffer,
c. blocking the well containing the immobilized biomolecule obtained in step (b) by loading a blocking agent into the well and heating the said well at a temperature ranging from 40-70 °C for a period ranging from 10-60 min and washing the said well with an appropriate washing buffer,
d. loading the corresponding antibody or antigen dissolved in a buffer into the well immobilized with antigen or antibody obtained in step (c) followed by heating the well at a temperature ranging from 40-80 °C for a period ranging from 10-70 min followed by washing with washing buffer,
e. loading an appropriate enzyme-conjugate dissolved in a suitable buffer into the well obtained in step (d) and heating the well at a temperature ranging from 40-80 °C for a period ranging from 20-70 min followed by washing with a washing buffer,
f. adding a substrate-dye-buffer to the well obtained in step (e) and keeping it for a period ranging from 4 to 10 min in dark followed by addition of stop solution and measuring optical density of the solution by spectrophotometer at a suitable wavelength;
wherein heating is carried out in an apparatus selected from laboratory polymerase chain reaction (PCR) thermocycler, specially designed thermocycler, incubator and water bath.

2. A method as claimed in claim 1 wherein the solid support used is made of a material selected from the group consisting of polycarbonate, polystyrene, polypropylene, polyethylene, glass, cellulose, nitrocellulose, silicagel and polyvinyl chloride.

3. A method as claimed in claim 2 wherein the solid support used is made of polycarbonate or polystyrene.

4. A method as claimed in claim 1 wherein the solid support is selected from the group consisting of PCR plates, ELISA plate, microwell plate, sheets, test particles selected from beads and microspheres, test tubes, test sticks, test strips, wells and modules.

5. A method as claimed in claim 1 wherein the activated solid support comprises a solid support capable of binding ligand molecules by covalent binding.

6. A method as claimed in claim 1 wherein the activated solid support is provided with an active functional group selected from the group consisting of halide, aldehyde, acetyl, epoxide, succinamide, isothiocyanate and acylazide for covalent binding.

7. A method as claimed in claim 6 wherein the active functional group is present in the solid support itself or is introduced by chemical or photochemical methods.

8. A method as claimed in claim 7 wherein the functional group is introduced on to the solid support by photochemical reaction using a photoactive compound selected from 4-azido-1-fluoro-2-nitrobenzene (1-fluoro-2-nitro-4-azidobenzene), N-hydroxysulfo-succinimidyl 4-azidobenzoate, N-hydroxysulfo-succinimidyl 4-azidosalicyclic acid and quinone or its derivatives.

9. A method as claimed in claim 3 wherein the polycarbonate or polystyrene support are activated by coating the support surface with 4-azido-1-fluoro-2-nitrobenzene and exposing the coated support to a light source to cause photoreaction.

10. A method as claimed in claim 9 wherein the light source is selected from UV lamp, laser beam and bright sunlight.

11. A method as claimed in claim 9 wherein the light source comprises UV radiation at 365 nm.

12. A method as claimed in claim 9 wherein the time for photoreaction for activation of solid support is in the range of 10 seconds to 10 hours.

13. A method as claimed in claim 1 wherein the assay is ELISA.

14. A method as claimed in claim 1 wherein the total time for antigen binding, blocking, antibody binding and conjugate binding is in the range of 2-6 h.

15. A method as claimed in claim 1 wherein in step (b) the buffer in which the antigen or antibody is dissolved is selected from the group consisting of carbonate buffer and phosphate buffer.

16. A method as claimed in claim 1 wherein in step (b) the buffer in which the antigen is dissolved has a pH in the range of from 6.5 to 11 with molarity ranging from 0.005 M to 0.1 M.

17. A method as claimed in claim 1 wherein the washing buffer used in step (b) comprises a mixture of phosphate buffer having a pH in the range of from 6.5 to 11, with molarity ranging from 0.005 M to 0.1 M and Tween 20 in the range of between 0.05% to 3%.

18. A method as claimed in claim 1 wherein the blocking agent is selected from the group consisting of bovine serum albumin, skimmed milk powder and gelatin.

19. A method as claimed in claim 1 wherein the antigen comprises a biomolecule or microorganism capable of eliciting an immune response.

20. A method as claimed in claim 1 wherein the conjugate in the enzyme conjugate of step (e) is selected from horseradish peroxidase and alkaline phosphatase.

21. A method as claimed in claim 1 wherein the enzyme in the enzyme conjugate is replaced by a label selected from chromophore and fluorophore.

22. A method as claimed in claim 1 wherein the immunosorbent assay is selected from a radio immunoassay, radio-immunosorbent test, radio allergosorbent test, biotin- avidin/streptavidin immunoassay, immunoblotting, immunostaining and ELISA.

23. A method as claimed in claim 22 wherein the ELISA is selected from the group consisting of direct ELISA, indirect ELISA and sandwich ELISA.

## Patentansprüche

1. Schnellverfahren für enzymgekoppelten Immunosorbens-Assay, **gekennzeichnet durch** die Verwendung eines aktivierten festen Trägers, wobei das Verfahren aufweist:
a. Bereitstellen eines aktivierten festen Trägers mit mindestens einer aktivierten Vertiefung,
b. Laden eines unter einem Antigen oder einem Antikörper ausgewählten Biomoleküls durch Auflösen des Biomoleküls in einem Beschichtungspuffer und Einbringen in die aktivierte Vertiefung des festen Trägers und Erhitzen der Vertiefung auf eine Temperatur im Bereich von 40-80°C während einer Zeitspanne im Bereich von 10-70 Minuten, und anschließendes gründliches Waschen der Vertiefung mit einem geeigneten Waschpuffer,
c. Blockieren der Vertiefung, die das im Schritt (b) erhaltene immobilisierte Biomolekül enthält, **durch** Laden eines Blockiermittels in die Vertiefung und Erhitzen der Vertiefung auf eine Temperatur im Bereich von 40-70°C während einer Zeitspanne von 10-60 Minuten und Waschen der Vertiefung mit einem geeigneten Waschpuffer,
d. Laden des entsprechenden, in einem Puffer aufgelösten Antikörpers oder Antigens in die im Schritt (c) erhaltene immobilisierte Vertiefung mit Antigen oder Antikörper und anschließendes Erhitzen der Vertiefung auf eine Temperatur im Bereich von 40-80°C während einer Zeitspanne von 10-70 Minuten, und anschließendes Waschen mit Waschpuffer,
e. Laden eines geeigneten Enzymkonjugats, das in einem geeigneten Puffer gelöst ist, in die im Schritt (d) erhaltene Vertiefung und Erhitzen der Vertiefung auf eine Temperatur im Bereich von 40-80°C während einer Zeitspanne von 20-70 Minuten, und anschließendes Waschen mit einem Waschpuffer,
f. Zugabe eines Substratfarbstoffpuffers in die im Schritt (e) erhaltene Vertiefung und Halten der Vertiefung im Dunkeln während einer Zeitspanne von 4 bis 10 Minuten gefolgt von Zugabe von Stoplösung, und Messen der Extinktion der Lösung mit einem Spektrophotometer bei einer geeigneten Wellenlänge;
wobei das Erhitzen in einer Vorrichtung erfolgt, die unter einem Labor-Polymerasekettenreaktions-Thermocycler (PCR-Thermocycler), einem speziell konstruierten Thermocycler, einem Inkubator und einem Wasserbad ausgewählt ist.

2. Verfahren nach Anspruch 1, wobei der verwendete feste Träger aus einem Material besteht, das aus der Gruppe ausgewählt ist, die aus Polycarbonat, Polystyrol, Polypropylen, Polyethylen, Glas, Cellulose, Nitrocellulose, Silicagel und Polyvinylchlorid besteht.

3. Verfahren nach Anspruch 2, wobei der verwendete feste Träger aus Polycarbonat oder Polystyrol besteht.

4. Verfahren nach Anspruch 1, wobei der feste Träger aus der Gruppe ausgewählt ist, die aus PCR-Platten, ELISA-Platten, Mikrotiterplatten, Folien, unter Perlen und Mikrokugeln ausgewählten Testteilchen, Teströhrchen, Teststäben, Teststreifen, Vertiefungen und Modulen besteht.

5. Verfahren nach Anspruch 1, wobei der aktivierte feste Träger einen festen Träger aufweist, der Ligandenmoleküle durch kovalente Bindung binden kann.

6. Verfahren nach Anspruch 1, wobei der aktivierte feste Träger mit einer aktiven funktionellen Gruppe ausgestattet wird, die aus der Gruppe ausgewählt ist, die aus Halogenid, Aldehyd, Acetyl, Epoxid, Succinamid, Isothiocyanat und Acylazid für kovalente Bindung besteht.

7. Verfahren nach Anspruch 6, wobei die aktive funktionelle Gruppe in dem festen Träger selbst vorhanden ist oder durch chemische oder photochemische Methoden eingebaut wird.

8. Verfahren nach Anspruch 7, wobei die funktionelle Gruppe in den festen Träger durch photochemische Reaktion unter Verwendung einer photoaktiven Verbindung eingebaut wird, die unter 4-Azido-1-fluor-2-nitrobenzol (1-Fluor-2-nitro-4-azidobenzol), N-Hydroxysulfosucccinimidyl-4-azidobenzoat, N-Hydroxysulfosucccinimidyl-4-azidosalicylsäure und Chinon oder ihren Derivaten ausgewählt ist.

9. Verfahren nach Anspruch 3, wobei der Polycarbonat- oder Polystyrolträger aktiviert wird, indem die Trägeroberfläche mit 4-Azido-1-fluor-2-nitrobenzol beschichtet und der beschichtete Träger mit einer Lichtquelle belichtet wird, um eine Photoreaktion auszulösen.

10. Verfahren nach Anspruch 9, wobei die Lichtquelle unter einer UV-Lampe, einem Laserstrahl und hellem Sonnenlicht ausgewählt ist.

11. Verfahren nach Anspruch 9, wobei die Lichtquelle UV-Strahlung bei 365 nm aufweist.

12. Verfahren nach Anspruch 9, wobei die Zeit für die Photoreaktion zur Aktivierung des festen Trägers im Bereich von 10 Sekunden bis 10 Stunden liegt.

13. Verfahren nach Anspruch 1, wobei der Assay der ELISA-Assay ist.

14. Verfahren nach Anspruch 1, wobei die Gesamtzeit zur Antigenbindung, Blockierung, Antikörperbindung und Konjugatbindung im Bereich von 2-6 Stunden liegt.

15. Verfahren nach Anspruch 1, wobei im Schritt (b) der Puffer, in dem das Antigen oder der Antikörper gelöst wird aus der Gruppe ausgewählt ist, die aus Carbonatpuffer und Phosphatpuffer besteht.

16. Verfahren nach Anspruch 1, wobei im Schritt (b) der Puffer, in dem das Antigen gelöst wird, einen pH-Wert im Bereich von 6,5 bis 11 bei einer Molarität im Bereich von 0,005 M bis 0,1 M aufweist.

17. Verfahren nach Anspruch 1, wobei der im Schritt (b) verwendete Waschpuffer ein Gemisch aus Phosphatpuffer mit einem pH-Wert im Bereich von 6,5 bis 11 bei einer Molarität im Bereich von 0,005 M bis 0,1 und Tween 20 in einem Anteil im Bereich von 0,05% bis 3% aufweist.

18. Verfahren nach Anspruch 1, wobei das Blockiermittel aus der Gruppe ausgewählt ist, die aus Rinderserumalbumin, Magermilchpulver und Gelatine besteht.

19. Verfahren nach Anspruch 1, wobei das Antigen ein Biomolekül oder einen Mikroorganismus aufweist, das bzw. der imstande ist, eine Immunantwort auszulösen.

20. Verfahren nach Anspruch 1, wobei das Konjugat in dem Enzymkonjugat von Schritt (e) unter Meerrettichperoxidase und alkalischer Phosphatase ausgewählt ist.

21. Verfahren nach Anspruch 1, wobei das Enzym in dem Enzymkonjugat durch einen Marker ausgetauscht wird, der unter einem Chromophor und einem Fluorophor ausgewählt ist.

22. Verfahren nach Anspruch 1, wobei der Immunosorbens-Assay unter einem Radioimmunoassay, Radioimmunosorbens-Test, Radioallergosorbens-Test, Biotin-Avidin/Strepavidin-Immunoassay, Immun-Blotting, Immunfärbung und ELISA ausgewählt ist.

23. Verfahren nach Anspruch 22, wobei der ELISA-Test aus der Gruppe ausgewählt ist, die aus direktem ELISA, indirektem ELISA und Sandwich-ELISA besteht.

## Revendications

1. Procédé rapide pour un dosage d'immunoadsorption enzymatique **caractérisé par** l'utilisation d'un support solide activé dans lequel ledit procédé comprend :
(a) la fourniture d'un support solide activé avec au moins un puits activé,
(b) la charge d'une biomolécule choisie parmi un antigène ou un anticorps par la dissolution de ladite biomolécule dans un tampon de revêtement et dans le puits activé du support solide, et le chauffage dudit puits à une température variant de 40 à 80 °C pendant une période variant de 10 à 70 minutes suivi d'un lavage minutieux du puits avec un tampon de lavage approprié,
(c) le blocage du puits contenant la biomolécule immobilisée obtenue à l'étape (b) par la charge d'un agent bloquant dans le puits et le chauffage dudit puits à une température variant de 40 à 70 °C pendant une période variant de 10 à 60 minutes, et le lavage dudit puits avec un tampon de lavage approprié,
(d) la charge de l'anticorps ou de l'antigène correspondant dissous dans un tampon, dans le puits immobilisé avec l'antigène ou l'anticorps obtenu à l'étape (c), suivi d'un chauffage du puits à une température variant de 40 à 80 °C pendant une période variant de 10 à 70 minutes, suivi d'un lavage avec un tampon de lavage,
(e) la charge d'un conjugué d'enzyme approprié dissous dans un tampon convenable, dans le puits obtenu à l'étape (d) et le chauffage du puits à une température variant de 40 à 80 °C pendant une période variant de 20 à 70 minutes, suivi d'un lavage avec un tampon de lavage,
(f) l'ajout d'un substrat-colorant-tampon au puits obtenu à l'étape (e) et son maintien pendant une période variant de 4 à 10 minutes dans l'obscurité, suivi de l'ajout d'une solution d'arrêt et la mesure de la densité optique de la solution à l'aide d'un spectrophotomètre à une longueur d'onde convenable ;
dans lequel le chauffage est réalisé dans un appareil choisi parmi un thermocycleur pour réaction en chaîne par polymérase (PCR) de laboratoire, un thermocycleur, un incubateur et un bain-marie spécialement conçus.

2. Procédé selon la revendication 1, dans lequel le support solide utilisé est réalisé dans un matériau choisi dans le groupe constitué par le polycarbonate, le polystyrène, le polypropylène, le polyéthylène, le verre, la cellulose, la nitrocellulose, le gel de silice et le polychlorure de vinyle.

3. Procédé selon la revendication 2, dans lequel le support solide utilisé est réalisé en polycarbonate ou polystyrène.

4. Procédé selon la revendication 1, dans lequel le support solide est choisi dans le groupe constitué par des plaques de PCR, une plaque ELISA, une plaque de micropuits, des feuilles, des particules tests choisies parmi les billes et les microsphères, des tubes à essai, des bâtonnets de test, des bandelettes d'essai, des puits et des modules.

5. Procédé selon la revendication 1, dans lequel le support solide activé comprend un support solide capable de lier des molécules de ligand par une liaison covalente.

6. Procédé selon la revendication 1, dans lequel le support solide activé est muni d'un groupe fonctionnel actif choisi dans le groupe constitué par un halogénure, un aldéhyde, un acétyle, un époxyde, un succinamide, un isothiocyanate et un acylazide pour une liaison covalente.

7. Procédé selon la revendication 6, dans lequel le groupe fonctionnel actif est présent dans le support solide lui-même ou bien est introduit par des procédés chimiques ou photochimiques.

8. Procédé selon la revendication 7, dans lequel le groupe fonctionnel est introduit sur le support solide par une réaction photochimique en utilisant un composé photoactif choisi parmi le 4-azido-1-fluoro-2-nitrobenzène (1-fluoro-2-nitro-4-azidobenzène), le 4-azidobenzoate de N-hydroxysulfo-succinimidyle, l'acide N-hydroxysulfo-succinimidyl 4-azidosalicylique et une quinone ou ses dérivés.

9. Procédé selon la revendication 3, dans lequel le support en polycarbonate ou polystyrène est activé par recouvrement de la surface du support avec du 4-azido-1-fluoro-2-nitrobenzène, et exposition du support recouvert à une source de lumière pour provoquer une photoréaction.

10. Procédé selon la revendication 9, dans lequel la source de lumière est choisie parmi une lampe UV, un faisceau laser et la lumière vive du soleil.

11. Procédé selon la revendication 9, dans lequel la source de lumière comprend un rayonnement UV à 365 nm.

12. Procédé selon la revendication 9, dans lequel le temps de photoréaction pour une activation du support solide est dans la gamme de 10 secondes à 10 heures.

13. Procédé selon la revendication 1, dans lequel le dosage est un ELISA.

14. Procédé selon la revendication 1, dans lequel le temps total de liaison de l'antigène, blocage, liaison de l'anticorps et liaison du conjugué est dans la gamme de 2 à 6 heures.

15. Procédé selon la revendication 1, dans lequel à l'étape (b) le tampon dans lequel l'antigène ou l'anticorps est dissous est choisi dans le groupe constitué par un tampon carbonate et un tampon phosphate.

16. Procédé selon la revendication 1, dans lequel à l'étape (b) le tampon dans lequel l'antigène est dissous possède un pH dans la gamme de 6,5 à 11 avec une molarité variant de 0,005 M à 0,1 M.

17. Procédé selon la revendication 1, dans lequel le tampon de lavage utilisé à l'étape (b) comprend un mélange de tampon phosphate ayant un pH dans la gamme de 6,5 à 11, avec une molarité variant de 0,005 M à 0,1 M et de Tween 20 dans la gamme entre 0,05 % et 3 %.

18. Procédé selon la revendication 1, dans lequel l'agent bloquant est choisi dans le groupe constitué par l'albumine issue du sérum de boeuf, la poudre de lait écrémé et la gélatine.

19. Procédé selon la revendication 1, dans lequel l'antigène comprend une biomolécule ou un microorganisme capable de susciter une réponse immune.

20. Procédé selon la revendication 1, dans lequel le conjugué dans le conjugué d'enzyme de l'étape (e) est choisi parmi la peroxydase de raifort et la phosphatase alcaline.

21. Procédé selon la revendication 1, dans lequel l'enzyme dans le conjugué d'enzyme est remplacée par une marque choisie parmi un chromophore et un fluorophore.

22. Procédé selon la revendication 1, dans lequel le dosage par immunoadsorption est choisi parmi un dosage radio-immunologique, un test par radio-immunoadsorption, un test par radio-allergoadsorption, un dosage immunologique par biotine-avidine/streptavidine, un transfert immunologique, une immunocoloration et un ELISA.

23. Procédé selon la revendication 22, dans lequel l'ELISA est choisi dans le groupe constitué par un ELISA direct, un ELISA indirect et un ELISA en sandwich.
